(19) Europäisches Patentamt
European Patent Office
Office européen des brevets

(11) Veröffentlichungsnummer: **0 298 314**
**A2**

(12) # EUROPÄISCHE PATENTANMELDUNG

(21) Anmeldenummer: **88110063.0**

(51) Int. Cl.4: **C07C 127/22 , A01N 47/34**

(22) Anmeldetag: **24.06.88**

(30) Priorität: **04.07.87 DE 3722155**

(43) Veröffentlichungstag der Anmeldung:
**11.01.89 Patentblatt 89/02**

(84) Benannte Vertragsstaaten:
**AT BE CH DE ES FR GB IT LI NL**

(71) Anmelder: **BASF Aktiengesellschaft**
**Carl-Bosch-Strasse 38**
**D-6700 Ludwigshafen(DE)**

(72) Erfinder: **Neubauer, Hans-Juergen, Dr.**
**B 4,2**
**D-6800 Mannheim 1(DE)**
Erfinder: **Hofmeister, Peter, Dr.**
**Bernard-Humblot-Strasse 12**
**D-6730 Neustadt(DE)**
Erfinder: **Kuenast, Christoph, Dr.**
**Salierstrasse 2**
**D-6701 Otterstadt(DE)**

(54) **(N-Benzoyl-N'-halogenalkoxycarbonylphenyl)-harnstoffe.**

(57) (N-Benzoyl-N′-halogenalkoxycarbonylphenyl)-harnstoffe der allgemeinen Formel I

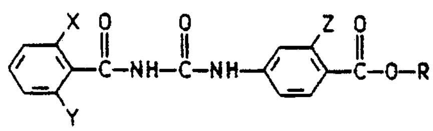

in der die Substituenten
R $C_1$-$C_{12}$-Halogenalkyl,
X Wasserstoff oder Halogen,
Y Halogen und
Z Wasserstoff oder Halogen bedeuten,
deren Herstellung und Verwendung zur Bekämpfung von Schädlingen.

EP 0 298 314 A2

## (N-Benzoyl-N′-halogenalkoxycarbonylphenyl)-harnstoffe

Die vorliegende Erfindung betrifft neue (N-Benzoyl-N′-halogenalkoxycarbonylphenyl)-harnstoffe der allgemeinen Formel I

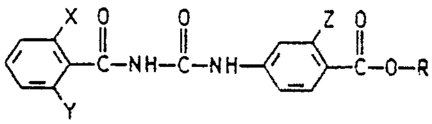

(I)

in der die Substituenten folgende Bedeutung haben:

R $C_1$-$C_{12}$-Halogenalkyl,
X Wasserstoff oder Halogen,
Y Halogen und
Z Wasserstoff oder Halogen.

Außerdem betrifft die Erfindung die Herstellung der Verbindungen I, Schädlingsbekämpfungsmittel, die die Verbindungen I als Wirkstoffe enthalten, sowie ein Verfahren zur Bekämpfung von Schädlingen.

Aus der DE-A-28 43 851 sind Verbindungen des Typs I als Insektizide bekannt, in denen der Rest R tert.-Alkyl bedeutet. Aus der EP-B-56 124 sind ferner Verbindungen des Typs I als insektizide Wirkstoffe bekannt, in denen der Rest R für einen primären oder sekundären $C_3$-$C_{12}$-Alkylrest steht. Die Wirkung dieser Verbindungen läßt vor allem bei niedrigen Aufwandmengen zu wünschen übrig.

Der Erfindung lag daher die Aufgabe zugrunde, neue (N-Benzoyl-N′-carbonylphenyl)-harnstoffe mit verbesserter Wirkung bereitzustellen.

Demgemäß wurden die eingangs definierten neuen (N-Benzoyl-N′-halogenalkoxycarbonylphenyl)-harnstoffe I, Verfahren zu deren Herstellung, Schädlingsbekämpfungsmittel, die die Verbindungen I als Wirkstoffe enthalten, sowie ein Verfahren zur Bekämpfung von Schädlingen mit (N-Benzoyl-N′-halogenalkoxycarbonylphenyl)-harnstoffen I gefunden.

Die Verbindungen I sind nach folgenden Methoden erhältlich:

a) durch Umsetzung eines Benzoylisocyanates der allgemeinen Formel II

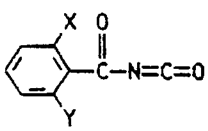

(II)

mit einem (p-Halogenalkoxycarbonyl)-anilin der allgemeinen Formel III

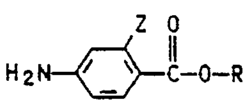

(III)

oder

b) durch Umsetzung eines Benzamides der allgemeinen Formel IV

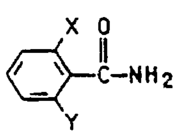

(IV)

mit einem (p-Halogenalkoxycarbonyl)-phenylisocyanat der allgemeinen Formel V

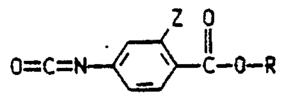 (V).

Die Umsetszung a) verläuft bei Temperaturen von 0 bis 120 °C, vorzugsweise 20 bis 80 °C und besonders bevorzugt bei 20 bis 60 °C, sowie bei Drücken zwischen 1 und 10 bar, vorzugsweise bei Normaldruck. Da die Reaktion unter Wärmeentwicklung (exotherm) verläuft, kann es von Vorteil sein, eine Kühlmöglichkeit vorzusehen.

Zweckmäßigerweise wird das (p-Halogenalkoxycarbonyl)-anilin der Formel III in einem Lösungs-oder Verdünnungsmittel vorgelegt unter anschließender Zugabe des Benzoylisocyanats der Formel II. Die Reaktion ist im allgemeinen nach 2 Stunden beendet.

Die Benzoylisocyanate II sind bekannt oder nach bekannten Methoden herstellbar (J. Org. Chem. 28, 1805-1811 (1963). Die (p-Halogen-alkoxycarbonyl)-aniline III sind gleichfalls bekannt oder nach bekannten Methoden herstellbar (Organikum, 9. Auflage, 1969, S. 446, 567, VEB Deutscher Verlag der Wissenschaften).

Die Umsetzung b) wird gegebenenfalls in Gegenwart eines Katalysators, bei Temperaturen von 0 bis 140 °C, vorzugsweise zwischen 60 und 100 °C, sowie bei Drücken zwischen 1 und 10 bar, vorzugsweise bei Normaldruck, durchgeführt. Die Reaktionsdauer liegt im allgemeinen zwischen 2 und 6 Stunden.

Als Katalysatoren eignen sich beispielsweise organische Basen, wie tert. Amine, z.B. Triethylamin, Pyridin, 4-N,N-Dimethylaminopyridin oder Alkylmetallverbindungen, wie Dibutylzinndiacetat,

Die Benzamide IV sind bekannt oder nach bekannten Methoden (z.B. Houben-Weyl, Methoden der organischen Chemie, Bd. E 5.2, S. 934 ff, Georg-Thieme Verlag, 1985) erhältlich. Die (p-Halogenalkoxycarbonyl)phenylisocyanate V sind z.T. bekannt oder sie können nach bekannten Methoden (z.B. Houben-Weyl, Methoden der organischen Chemie, Bd. E 4, S. 738 ff, Georg Thieme Verlag, 1983) erhalten werden.

Üblicherweise setzt man die Ausgangsstoffe II und III bzw. IV und V in stöchiometrischem Verhältnis ein. Ein Überschuß der einen oder anderen Komponente kann in Einzelfällen aber durchaus vorteilhaft sein.

Die Umsetzungen a) und b) werden zweckmäßigerweise in einem Lösungs- oder Verdünnungsmittel ausgeführt. Hierzu sind beispielsweise geeignet; aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlormethan und Chlorbenzol, Ether oder Ester wie Diethyl- und Di-n-Butylelther, Methyl-tert.-butylether, Tetrahydrofuran, Dioxan und Essigsäureethylester; Ketone, beispielsweise Aceton, Methylethylketon und Methylisopropylketon; ferner Nitrile, wie Acetonitril und Propionitril; aprotische dipolare Lösungsmittel, wie Dimethylformamid, Dimethylsulfoxid oder Pyridin. Auch Gemische dieser Stoffe können als Lösungs- oder Verdünnungsmittel verwendet werden.

Die neuen Verbindungen der Formel I fallen teilweise in Form von Ölen an, die sich durch längeres Erwärmen unter vermindertem Druck auf mäßig erhöhte Temperatur ("Andestillieren") von den letzten flüchtigen Anteilen befreien und auf diese Weise reinigen lassen. Erhält man die Verbindungen der Formel I in kristalliner Form, so kann ihre Reinigung durch Umkristallisation erfolgen.

Im einzelnen haben die Substituenten in Formel I folgende Bedeutungen:

R - verzweigte und unverzweigte $C_1$-$C_{12}$-Halogenalkyl, bevorzugt verzweigte und unverzweigte $C_3$-$C_{12}$-Halogenalkyl, besonders bevorzugt verzweigte und unverzweigte $C_3$-$C_8$-Halogenalkyl, wobei der Alkylrest 1 oder mehrere, z.B. 1 bis 9, insbesondere 1 bis 7 Halogenatome enthält und unter den Halogenalkylen bevorzugt Chloralkyl, wie 1-(Chlormethyl)-ethyl und Bis-chlormethyl-methyl, besonders bevorzugt Fluoralkyl, wie 3-Fluor-n-propyl, 2,2,3,3-Tetrafluor-n-propyl, 2,2,3,3,4,4,4-Heptafluor-n-butyl, 1-(Fluormethyl)-ethyl, 1-(Trifluormethyl)-ethyl, Bis-fluormethyl-methyl und 1,1-Di-trifluormethyl-ethyl.

X - Wasserstoff,
- Halogen, bevorzugt Fluor, Chlor und Brom, besonders bevorzugt Fluor und Chlor,

Y - Halogen, bevorzugt Fluor, Chlor und Brom, besonders bevorzugt Fluor und Chlor,

Z - Wasserstoff,
- Halogen, bevorzugt Fluor und Chlor, besonders bevorzugt Chlor.

Die (N-Benzoyl-N'-halogenalkoxycarbonylphenyl)-harnstoffe der allgemeinen Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Im Gegensatz zu den meisten bisher bekannten Wirkstoffen, die als Kontakt- oder Fraßgifte die Tiere töten, lähmen oder vertreiben, wirken die Verbindungen der Formel I entwicklungsstörend auf den tierischen Organismus. Bei Insekten wird beispielsweise die Umwandlung zur Imago, die Ablage von entwicklungsfähigen Eiern und die Entwicklung von abgelegten normalen Eiern gestört und dadurch die Generationsfolge unterbrochen. Für Wirbeltiere sind die erfindungsgemäßen Wirkstoffe praktisch ungiftig. Die Verbindungen der Formel I werden überdies leicht zu Stoffen abgebaut, die in der natürlichen Umgebung vorkommen und von Mikroorganismen weiter zerlegt werden. Die Gefahr einer Kumulation ist deshalb ausgeschlossen. Sie können demgemäß unbedenklich zur Bekämpfung von Schädlingen bei Tieren, Pflanzen und Vorräten eingesetzt werden.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaum gespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea ptiyocampa (Pinienprozessionsspinner), Phalera bucephala (Mondfleck), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagai (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-puncta (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Ortiorrhynchus sulcatus (Gefurchter Lappenrüßler), Otiorrhynchus ovatus (Erdbeerwurzelrüßler), Hylobius abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);

aus der Ordnung der Zweiflügler (Diptera) beispielsweise Lycoria pectoralis, Mayetiola destructor (Hessenfliege), Basineura brassicae (Kohlschoten-Gallmücke), Contarinia tritici (Gelbe Weizen-Gallmücke), Haplodiplosis equestris (Sattelmücke), Tipula paludosa (Weisenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hysocyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ovis, Chrysomya macellaria, Chrysomya hominivorax, Lucilia cuprina, Lucilia sericata, Hypoderma lineata;

aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), Atta sexdens (Blattschneiderameise);

aus der Ordnung der Wanzen (Heteroptera) beispielsweise Nezara viridula (Grüne Reiswanze), Eurygaster integriceps (Asiatische Getreidewande), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nasturtii (Kreuzdornblattlaus), Cerosipha gossypii (Gurkenblattlaus), Sappaphis mali (Rosige Apfellaus), Sappaphis mala (Mehlige Birnblattlaus), Dysaphis radicola (Mehlige Apfel-faltenlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkrischenlaus), Dysaulacorthum pseudosolani (Gefleckte Kartoffellaus), Acyrthosiphon onobrychis (Grüne Erbsenalaus), Macrosiphon rosae (Große Rosenblattlaus), Megoura viciae (Wickenlaus) Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);
aus der Ordnung der Termiten (Isoptera) beispielsweise Reticulitermes lucifugus, Calotermes flavicollis, Leucotermes flavipes, Termes natalensis;
aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melanoplus femur-rubrum (Rotbeinige Heuschrecke), Blatta orientalis (Küchenschabe), Blattella germanica (Deutsche Schabe), Periplaneta americana (Amerikanische Schabe), Blabera gigantes (Riesenschabe).

Zur Klasse der Arachnoidea gehören Spinntentiere (Acarina) beispielsweise Ixodes ricinus (Holzbock), Ornithodorus moubata, Amblyomma americanum, Dermacentor silvarum, Boophilus microplus, Tetranychus telarius, Tetranychus pacificus, Paratetranychus pilosus, Bryobia praetiosa.

Zur Klasse der Nematoden zählen beispielsweise Wurzelgallennematoden, z.B. Meloidogyne incognita, Meloidogyne hapla, Meloidogyne javanica, Zysten bildende Nematoden, z.B. Globodera rostochiensis, Heterodera schachtii, Heterodera avenae, Heterodera glycinae, Heterodera trifolii, Stock- und Blattälchen, z.B. Ditylenchus dipsaci, Ditylenchus destructor, Pratylenchus neglectus, Pratylenchus penetrans, Pratylenchus goodeyi, Paratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwendungsmöglichkeiten, z.B. in Form direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungs zwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können als Emulsionskonzentrate, Pasten oder netzbare Pulver (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkali, Salze sulfatierter Hexadecanole, Heptadecanole, Octadecanole, Salze von sulfatiertem Fettalkoholglykolether, Kondensationsprodukt von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Aklylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr. 5 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 6 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 10 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 7 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 11 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden.

Im allgemeinen liegen sie zwischen 0,0001 und 10 Gew.%, vorzugsweise zwischen 0,01 und 1 Gew.%. Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,001 bis 10, vorzugsweise 0,02 bis 0,2 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Beispielsweise können folgende Mittel zugemischt werden: 1,2-Dibrom-3-chlorpropan, 1,3-Dichlorpropen, 1,3-Dichlorpropen + 1,2-Dichlorpropan, 1,2-Dibromethan, 2-sec.-Butyl-phenyl-N-methylcarbamat, o-Chlorphenyl-N-methylcarbamat, 3-Isopropyl-5-methylphenyl-N-methylcarbamat, o-Isopropoxyphenyl-N-methylcarbamat, 3,5-Dimethyl-4-methylmercapto-phenyl-N-methylcarbamat, 4-Dimethylamino-3,5-xylyl-N-methylcarbamat, 2-(1,3-Dioxolan-2-yl)-phenyl-N-methylcarbamat, 1-Naphthyl-N-methylcarbamat, 2,3-Dihydro-2,2-dimethyl-benzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-O-(methylcarbomoyl)-oxim, S-Methyl-N[(methylcarbamoyl)-oxyl]-thio-acetimidat, Methyl-N′,N′-dimethyl-N-[(methylcarbomoyl)oxy]-1-thiooxamidat, N-(2-Methylchlorphenyl)-N′,N′-dimethylformamidin, Tetrachlorthiophen, 1-(2,6-Difluor-benzoyl)-3-(4-chlorphenyl)-harnstoff, O,O-Dimethyl-O-(p-nitrophenyl)-phosphorthioat, O,O-Diethyl-O-(p-nitrophenyl)-phosphorthioat, O-Ethyl-O(p-nitrophenyl)-phenyl-phosphonothioat, O,O-Dimethyl-O-(3-methyl-4-nitrophenyl)-phosphorthioat, 0,0-Diethyl-O-(2,4-dichlorphenyl)-phosphorthioat, O-Ethyl-O-(2,4-dichlorphenyl)-phenyl-phosphonothioat, O,O-Dimethyl-O-(2,4,5-trichlorphenyl)-phosphorthioat, O-Ethyl-O-(2,4,5-trichlorphenyl)-ethyl-phosphorthioat, O,O-Dimethyl-O-(4-brom-2,5-dichlorphenyl)-phosphorthioat, O,O-Dimethyl-O-(2,5-dichlor-4-jodphenyl)-phosphorthioat, O,O-Dimethyl-O-(3-methyl-4-methylthiophenyl)-phosphorthioat, O-Ethyl-O-(3-methyl-4-methylthiophenyl)-isopropyl-phosphoramidat, O,O-Diethyl-O-(p-methylsulfinyl-phenyl)-phosphorthioat, O-Ethyl-S-phenyl-ethyl-phosphonodithioat, O,O-Diethyl-[2-chlor-1-(2,4-dichlorphenyl)-vinyl]-phosphat, O,O-Dimethyl-[2-chlor-1-(2,4,5-trichlorphenyl]-vinyl- phosphat, O,O-Dimethyl-S-(1′-phenyl)-ethylacetat-phosphordithioat, Bis-(dimethylamino)-fluorphosphinoxid, Octamethyl-pyrophosphoramid, O,O,O,O-Tetraethyldithio-pyrophosphat, S-Chlormethyl-O,O-diethyl-phosphordithioat, O-Ethyl-S,S-dipropyl-phosphordithioat, O,O-Dimethyl-O-2,2-dichlorvinyl-phosphat, O,O-Dimethyl-1,2-dibrom-2,2-dichlorethylphosphat, O,O-Dimethyl-2,2,2-trichlor-1-hydroxy-ethylphosphonat, O,O-Dimethyl-S-[1,2-biscarbethoxy-ethyl-(1)]-phosphordithioat, O,O-Dimethyl-O-(1-methyl-2-carbmethoxy-vinyl)-phosphat, O,O-Dimethyl-S-(N-methyl-carbamoylmethyl)-phosphordithioat, O,O-Dimethyl-S-(N-methylcarbamoyl-methyl)-phosphorthioat, O,O-Dimethyl-S-(N-methoxyethyl-carbamoylethyl)-phosphordithioat, O,O-Dimethyl-S-(N-formyl-N-methyl-carbamoyl-methyl)-phosphordithioat, O,O-Dimethyl-O-[1-methyl-2-(methyl-carbamoyl)-vinyl]-phosphat, O,O-Dimethyl-O-[1-methyl-2-dimethylcarbamoyl)-vinyl]-phosphat, O,O-Dimethyl-O-[(1-methyl-2-chlor-2-diethylcarbamoyl)-vinyl]-phosphat, O,O-Diethyl-S-(ethylthiomethyl)-phosphordithioat, O,O-Diethyl-S[(p-chlorphenylthio)-methyl]-phosphordithioat, O,O-Dimethyl-S-(2-ethylthioethyl)-phosphorthioat, O,O-Dimethyl-S-(2-ethylthioethyl)-phosphordithioat, O,O-Dimethyl-S-(2-ethylsulfinyl-ethyl)-phosphorthioat, O,O-Diethyl-S-(2-ethylthio-ethyl)-phosphordithioat, O,O-Diethyl-S-(2-ethylsulfinyl-ethyl)-phosphorthioat, O,O-Diethyl-thiophosphoryliminophenyl)-acetonitril, O,O-Diethyl-S-(2-chlor-1-phthalimidoethyl)-phosphordithioat, O,O-Diethyl-S-[6-chlor-benzoxazolon-(2)-yl(3)]-methyldithiophosphat, O,O-Dimethyl-S-[2-methoxy-1,3,4-thiadiazol-5-[4H]-onyl-(4)-methyl]-phosphordithioat, O,O-Diethyl-O-[3,5,6-trichlor-pyridyl-(2)]-phosphorthioat, O,O-Diethyl-O-(2-pyrazinyl)-phosphorthioat, O,O-Diethyl-O-[2-isopropyl-4-methylpyrimidinyl(6)]-phosphorthioat, O,O-Diethyl-O-[2-(diethylamino)-6-methyl-4-pyrimidinyl]-thionophosphat, O,O-Dimethyl-S-(4-oxo-1,2,3-benzotriazin-3-[4H]-yl-methyl)-phosphordithioat, O,O-Dimethyl-S-[4,6-diamino-1,3,5-triazin-2-yl)-methyl]-phosphordithioat, O,O-Diethyl-(1-phenyl-1,2,4-triazol-3-yl)-thionophosphat, O,S-Dimethyl-phosphor-amidothioat, O,S-Dimethyl-N-acetyl-phosphoramidothioat, alpha-Hexachlorcyclohexan, 1,1-Di-(p-methoxyphenyl)-2,2,2-trichlor-ethan, 6,7,8,9,10,10-Hexachloro-1,5,5a,6,9,9a-hexahydro-6,9-methano-2,4,3-benzodioxa-thiepin-3-oxid, Pyrethrine, DL-2-Allyl-3-methyl-cyclopenten-(2)-on-(1)-yl-(4)-DL-cis,-trans-chrysanthemat, 5-Benzyl-furyl-(3)-methyl-DL-cis,-trans-chrysanthemat, 3-Phenoxybenzyl(±)-cis,-trans-2,2-dimethyl-3-(2,2-dichlorvinyl)-cyclopropancarboxylat, alpha-Cyano-3-phenoxybenzyl(±)-cis,-trans-2,2-dimethyl-3-(2,2-dichlorvinyl)-cyclopropancarboxylat, (s)-alpha-Cyano-3-phenoxybenzyl-cis(1R,3R)-2,2-dimethyl-3-(2,2-dibromvinyl)-cyclopropancarboxylat, 3,4,5,6-Tetrahydrophthalimidoethyl-DL-cis,-trans-chrysanthemat, 2-Methyl-5-(2-propinyl)-3-furylmethyl-chrysanthemat, (alpha-Cyano-3-phenoxybenzyl)-alpha-isopropyl-4-chlorphenylacetat.

## Herstellungsbeispiel

N-[3-Chlor-4-(1,1-di-trifluormethyl-ethoxycarbonyl)-phenyl]-N′-(2,6-difluorbenzoyl)-harnstoff (Verbindung Nr. 5)

Eine Lösung von 12,5 g 2-Chlor-4-nitro-benzoesäurechlorid in 150 ml abs. Pyridin wird auf 0 °C abgekühlt und tropfenweise mit 11,8 g Hexafluor-2-methyl-propan-2-ol versetzt. Man läßt noch 8 Stunden bei Raumtemperatur rühren, gießt das Reaktionsgemisch in 100 ml Eiswasser und stellt mit konz. Salzsäure auf pH 2 ein. Die wäßrige Phase wird dreimal mit Ether extrahiert und die vereinigten Etherextrakte mit Natriumhydrogencarbonatlösung und Wasser gewaschen. Nach Trocknung über Natriumsulfat und Entfernen des Lösungsmittels i. Vak. erhält man 16,5 g (79 %) 2-Chlor-4-nitro-benzoesäure-(1,1-di-trifluormethyl)-ethylester.

15,8 g dieses Produkts werden in 100 ml abs. Tetrahydrofuran bei 1 bar $H_2$-Druck über 1 g Raney-Nickel bei Raumtemperatur hydriert. Man trennt vom Katalysator ab, engt ein und erhält 14,2 g (98 %) 4-Amino-2-chlorbenzoesäure-(1,1-di-trifluormethyl)-ethylester.

Zu einer Lösung von 4,7 g des zuvor erhaltenen Produkts in 50 ml wasserfreiem Tetrahydrohuran tropft man bei Raumtemperatur 2,8 g 2,6-Difluorbenzoylisocyanat. Anschließend wird 2 h bei 40 °C gerührt, mit 100 ml Pentan versetzt und das ausgefallene Produkt abgesaugt. Nach Trocknung über Natriumsulfat erhält man 5,0 g N-[3-Chlor-4-(1,1-ditrifluormethyl-ethoxycarbonyl)-phenyl]-N′-(2,6-difluorbenzoyl)-harnstoff

(Verbindung Nr.5) in 69 %iger Ausbeute; Fp. 178 bis 180° C.

Die in der nachstehenden Tabelle aufgeführten Verbindungen I können nach dem erfindungsgemäßen Verfahren aus entsprechenden Vorprodukten ohne weiteres erhalten werden und lassen eine gleichartige Wirkung erwarten.

Tabelle

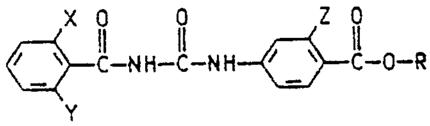

(I)

| Verbindung Nr. | X | Y | Z | R | Schmelzpunkt [°C] |
|---|---|---|---|---|---|
| 1 | F | F | Cl | 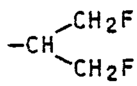 | 184 - 186 |
| 2 | Cl | Cl | Cl | " | 205 - 207 |
| 3 | F | F | H | " | |
| 4 | Cl | H | H | " | |
| 5 | F | F | Cl | 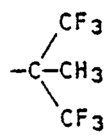 | 178 - 180 |
| 6 | F | F | H | " | 225 - 227 |
| 7 | Cl | Cl | Cl | " | 173 - 175 |
| 8 | Cl | Cl | H | " | |
| 9 | Cl | H | Cl | " | |
| 10 | Cl | H. | H | " | 153 - 155 |
| 11 | F | F | Cl | 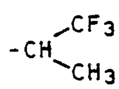 | 188 - 191 |
| 12 | Cl | Cl | Cl | " | 204 - 206 |
| 13 | Cl | H | Cl | " | |
| 14 | F | F | H | " | |
| 15 | Cl | Cl | H | " | |
| 16 | Cl | H | H | " | |

| Verbindung Nr. | X | Y | Z | R | Schmelzpunkt [°C] |
|---|---|---|---|---|---|
| 17 | F | F | Cl | 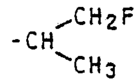 | 160 - 163 |
| 18 | F | F | H | " | |
| 19 | Cl | Cl | Cl | " | 181 - 183 |
| 20 | Cl | Cl | H | " | |
| 21 | Cl | H | Cl | " | |
| 22 | Cl | H | H | " | |
| 23 | F | F | Cl | $-CH_2-CH_2-CH_2-F$ | 163 - 165 |
| 24 | F | F | H | " | |
| 25 | Cl | Cl | Cl | " | 157 - 158 |
| 26 | Cl | Cl | H | " | |
| 27 | Cl | H | Cl | " | |
| 28 | Cl | H | H | " | |
| 29 | F | F | Cl | $-CH_2-CF_2-CHF_2$ | 184 - 187 |
| 30 | F | F | H | " | |
| 31 | Cl | Cl | Cl | " | 172 - 174 |
| 32 | Cl | Cl | H | " | |
| 33 | Cl | H | Cl | " | |
| 34 | Cl | H | H | " | |
| 35 | F | F | Cl | $-CH_2-CF_2-CF_2-CF_3$ | 166 - 169 |
| 36 | F | F | H | " | |
| 37 | Cl | Cl | Cl | " | 121 - 123 |
| 38 | Cl | Cl | H | " | |
| 39 | Cl | H | Cl | " | |
| 40 | Cl | H | H | " | |
| 41 | F | F | Cl | 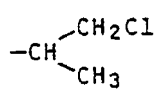 | 156 - 158 |
| 42 | F | F | Cl | 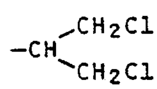 | 156 - 159 |

Anwendungsbeispiele

In den folgenden Beispielen wurden die erfindungsgemäßen Verbindungen bzw. sie enthaltende Mittel hinsichtlich ihrer Wirkung auf Schädlinge mit den nachstehenden Verbindungen des Standes der Technik verglichen.

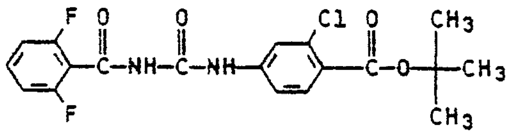

bekannt aus
DE-A-28 43 851 als
Verbindung Nr. 22

Die Konzentrationen, bei denen die untersuchten Verbindungen eine 100 %ige Mortalität bzw. Hemmung bewirken, sind die jeweiligen Minimalkonzentrationen. Bei jeder Konzentration wurden mindestens 2 Becher angesetzt.

Beispiel A

Zuchtversuch mit Dysdercus intermedius (Baumwollwanzen)

Petrischalen von 10 cm Durchmesser werden mit 1 ml der acetonischen Wirkstofflösung ausgekleidet. Nach Verdunsten des Lösungsmittels besetzt man die Schalen mit je 20 Larven des vorletzten Stadiums. Nach 24 Stunden überführt man die überlebenden Tiere in 1 l-Gläser, die 200 g sterilen Quarzsand (Korngröße 0 bis 3 mm) enthalten. Dieser Sand wurde vor Versuchsbeginn mit 25 ml der wäßrigen Wirkstoffaufbereitung angegossen. Als Futter bietet man gequollene Baumwollsamen, die wöchentlich gewechselt werden. Ebenfalls wöchentlich feuchtet man den Sand mit reinem Wasser an.

Die Versuchstemperatur beträgt 25 bis 27 °C. Die Beobachtungszeit erstreckt sich bis zum Schlüpfen der Eier in den Kontrollen.

In diesem Versuch lag die mortale Dosis der Verbindung Nr. 5 bei 4 ppm, bei der Vergleichsverbindung A bei 5 ppm.

Beispiel B

Zuchtversuch mit Aedes aegypti (Gelbfiebermücken)

Bei 25 °C werden in einem 250 ml Plastikbecher 200 ml Leitungswasser mit der Wirkstoffaufbereitung versetzt und anschließend mit 20 bis 30 Moskitolarven im dritten bis vierten Larvenstadium besetzt. Während der Versuchsdauer wird einmal mit einem gepulverten, käuflichen Zierfischfutter (Tetramin®) gefüttert. Beobachtet werden Verpuppung und Schlupf der Imagines. Dies erfolgt nach ca. 10 bis 12 Tagen.

Bei diesem Versuch lag die mortale Dosis der Verbindungen 5, 6 und 10 bei 0,004 ppm, die der Verbindungen 7, 12 und 19 bei 0,1 ppm, die der Verbindungen 11 und 41 bei 0,4 ppm und die Vergleichsverbindung A zeigte bei 1 ppm keine Wirkung.

Beispiel C

Entwicklungshemmung von Prodenia litura (Ägyptischer Baumwollwurm) auf behandeltem Nährboden

Ein 250 ml Plastikbecher wird mit ca. 100 ml des Standard-Nährbodens angefüllt, der mit einer flüssigen Wirkstoffaufbereitung sorgfältig durchmischt ist. Bei der Versuchstemperatur von 25 bis 26 °C werden je Konzentration mindestens 2 Becher angesetzt, von denen jeder mit 5 Raupen im dritten Larvenstadium (Länge ca. 10 bis 12 mm) besetzt wird. Die Gefäße werden mit perforierten, transparenten Deckeln verschlossen und bis zum Schlupf der Falter beobachtet.

Bei diesem Versuch lag die mortale Dosis der Verbindung 6 bei 0,2 ppm, die der Verbindung 10 bei 0,4 ppm.

Beispiel D

Chemosterilisation von Musca domestica (Stubenfliegen)

Ca. 100 schlüpffähige Puppen von Stubenfliegen werden in Folienbehälter mit 8,5 l Volumen gesetzt. Sie erhalten hier 2 g Futter, bestehend aus
6 Teilen Kristallzucker
6 Teilen Trockenmilch
1 Teil Eipulver
dem zunächst 20 mg des Wirkstoffes in organischer Lösung zugefügt wurden (= 1 %). Wasser wird in ausreichender Menge auf Zellstoff in 100 ml Kunststoffbechern geboten. Die Tiere bleiben ca. 8 Tage in diesen Käfigen bei 22 °C im Tag- und Nachtrhythmus. Am 8. Tag gibt man ein Eiablagengefäß mit in Magermilch getränktem Zellstoff in die Käfige. Hier erfolgt innerhalb der nächsten 24 Stunden die Eiablage. Beim Einstellen der Eiablagegefäße wird die Fraßgiftwirkung der toten Fliegen beurteilt. Nach 3 bis 4 Tagen wird die Entwicklung der Eier auf die Milchwatte beurteilt.

Eine 80 %ige Mortalität war bei den Verbindungen 5, 6 und 10 bei einer Dosis kleiner oder gleich 0,02 Gew. % festzustellen.

Beispiel E

Zuchtversuch von Ostrinia nubilalis (Maiszünsler)

Je 50 ml eines Nährbodens folgender Zusammensetzung:
515 g Maismehl
130 g Weizenkeime
137 g Bierhefe
18 g Ascorbinsäure
10 g Cellulosepulver
5 g Nipagin
20 g Wessons Salz
20 ml Vitaminlösung
80 g Agar
3100 ml Wasser
füllt man in 100 ml Plastikbecher und mischt die wäßrige Wirkstoffaufbereitung sorgfältig unter. Nach dem Erkalten belegt man jedes Gefäß mit 4 Raupen des dritten Larvenstadiums. Pro Konzentration werden 5 Gefäße angesetzt. Die Beobachtung erstreckt sich bis zum Schlüpfen der Falter.

Bei diesem Versuch lag die mortale Dosis der Verbindung 5 bei 10 ppm, die der Verbindung 12 bei 4 ppm und eine 80 %ige Mortalität erzielte man mit Verbindung 11 bei 0,4 ppm.

Beispiel F

Zuchtversuch von Musca domestica (Stubenfliegen)

Als Versuchsgefäße dienen 50 ml Gläser. Diese werden mit je 4,75 ml Nährbrei beschickt und mit 0,25 ml wäßriger Wirkstoffaufbereitung versetzt. In einem Becherglas schwemmt man ca. 10 000 einen Tag alte Fliegeneier in ca. 100 ml Leitungswasser auf. Auf dieser Suspension überträgt man mittels einer Pipette je einen Tropfen in ein Glas. Der fertige Versuchsansatz wird mit einem Wattepfropfen verschlossen und bei 23 bis 24 °C gelagert. Am 5 . Tag erfolgt die Auswertung.

Bei diesem Versuch wurde eine 80 %ige Mortalität mit Verbindung 6 bei 4 ppm oder mit Verbindung 37 bei 20 ppm erzielt, wohingegen 50 ppm der Vergleichsverbindung A keine Wirkung zeigte.

## Ansprüche

1. (N-Benzoyl-N′-halogenalkoxycarbonylphenyl)-harnstoffe der allgemeinen Formel I

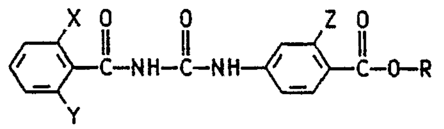

(I),

in der die Substituenten folgende Bedeutung haben:
R $C_1$-$C_{12}$-Halogenalkyl,
X Wasserstoff oder Halogen,
Y Halogen und
Z Wasserstoff oder Halogen.

2. (N-Benzoyl-N′-halogenalkoxycarbonylphenyl)-harnstoffe der Formel I nach Anspruch 1, in der die Substituenten folgende Bedeutung haben:
R $C_3$-$C_{12}$-Halogenalkyl,
X Wasserstoff, Fluor oder Chlor,
Y Fluor oder Chlor und
Z Wasserstoff oder Chlor.

3. Verfahren zur Herstellung von (N-Benzoyl-N′-halogenalkoxycarbonylphenyl)-harnstoffen der allgemeinen Formel I gemäß Anspruch 1, dadurch gekennzeichnet, daß man
a) ein Benzoylisocyanat der allgemeinen Formel II

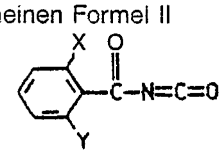

(II)

mit einem (p-Halogenalkoxycarbonyl)-anilin der allgemeinen Formel III

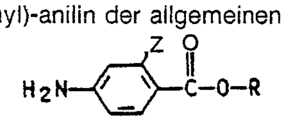

(III)

b) ein Benzamid der allgemeinen Formal IV

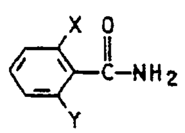

(IV)

mit einem (p-Halogenalkoxycarbonyl)-phenylisocyanat der allgemeinen Formel V

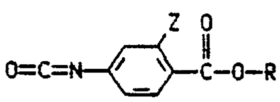

(V)

in an sich bekannter Weise umsetzt.

4. Schädlingsbekämpfungsmittel, enthaltend einen (N-Benzoyl-N′-halogenalkoxycarbonylphenyl)-harnstoff der Formel I gemäß Anspruch 1 neben üblichen Trägerstoffen.

5. Schädlingsbekämpfungsmittel gemäß Anspruch 4, dadurch gekennzeichnet, daß es 0,1 bis 95 Gew.% eines (N-Benzoyl-N′-halogenalkoxycarbonylphenyl)- harnstoffes 1 enthält.

6. Verfahren zur Bekämpfung von Schädlingen, dadurch gekennzeichnet, daß man die Schädlinge und/oder die von Schädlingen freizuhaltenden Flächen und/oder Räume mit einer für Schädlinge wirksamen Menge eines (N-Benzoyl-N′-halogenalkoxycarbonylphenyl)-harnstoffes der Formel I gemäß Anspruch 1 behandelt.